# EUROPEAN PATENT SPECIFICATION

(11) **EP 2 380 573 B1**
(45) Date of publication and mention of the grant of the patent: **25.02.2015**
(21) Application number: 11174403.3
(22) Date of filing: 06.05.2005
(51) Int. Cl.: A61K 31/55, A61P 29/00

(54) **Eslicarbazepine acetate and methods of use**
Eslicarbazepinacetat und Verwendungsverfahren
Acétate d'eslicarbazépine et procédés d'utilisation

(43) Date of publication of application: 26.10.2011
(62) Divisional of application: 05740623.3
(73) Proprietor: Bial-Portela & CA, S.A., 4745-457 S. Mamede do Coronado (PT)
(72) Inventor: Vieira Araujo Soares da Silva, Patrício Manuel, 4150 Porto (PT); de Almeida, José Luís, P-4540-273 Arouca (PT)
(74) Representative: Curtis, Philip Anthony

(56) References cited:
- EP-A1- 1 477 480
- WO-A1-03/042182
- WO-A1-2004/071513
- US-A1- 2004 038 874
- HAINZL D ET AL: "METABOLISM OF TWO NEW ANTIEPILEPTIC DRUGS AND THEIR PRINCIPAL METABOLITES S(+)- AND R(-)-10,11-DIHYDRO-10-HYDROXY CARBAMAZEPINE", EPILEPSY RESEARCH, ELSEVIER SCIENCE PUBLISHERS, AMSTERDAM, NL, vol. 44, no. 2/03, 1 May 2001 (2001-05-01) , pages 197-206, XP001181474, ISSN: 0920-1211, DOI: 10.1016/S0920-1211(01)00231-5
- CARRAZANA E ET AL: "RATIONALE AND EVIDENCE FOR THE USE OF OXCARBAZEPINE IN NEUROPATHIC PAIN", JOURNAL OF PAIN AND SYMPTOM MANAGEMENT, ELSEVIER, NEW YORK, NY, US, vol. 25, no. 5, SUPPL, 1 May 2003 (2003-05-01), pages S31-S35, XP009042848, ISSN: 0885-3924, DOI: 10.1016/S0885-3924(03)00067-8

## Description

### BACKGROUND

The present disclosure relates to a pharmaceutical composition and a treatment method using eslicarbazepine acetate and in particular to the use of eslicarbazepine acetate in the manufacture of a pharmaceutical composition for treating post-herpetic neuralgia.

Epilepsy, pain conditions such as trigeminal neuralgia, and affective brain disorders such as bipolar disorder are commonly treated with carbamazepine. Treatment with carbamazepine, however, can lead to serious side effects due to the production of toxic metabolites. Oxcarbazepine was developed to reduce the severity of those side effects, but oxcarbazepine has a greatly reduced potency. *See*, *e.g*., Almeida, L. & Soares-da-Silva, P., "Safety, Tolerability, and Pharmacokinetic Profile of BIA 2-093, a Novel Putative Antiepileptic, in a Rising Multiple-Dose Study in Young Healthy Humans," J. Clin. Pharmacol., 44, 906-918 (2004) (herein referred to as *"Almeida I*").

Thus, there is a need for a pharmaceutical composition and method for treating various conditions or diseases such as, for example, epilepsy, trigeminal neuralgia, and affective brain disorders, that has a high potency and a low occurrence of side effects.

### SUMMARY

Eslicarbazepine acetate, (S)-(-)-10-acetoxy-10,11-dihydro-5H-dibenz/b,f/azepine-5-carboxamide ("BIA 2-093"), is a new drug currently being developed which is useful for the treatment of various conditions, such as, for example, epilepsy and affective brain disorders, as well as pain conditions and nervous function alterations in degenerative and post-ischemic diseases. Although chemically related to carbamazepine and oxcarbazepine, eslicarbazepine acetate is believed to avoid the production of certain toxic metabolites (such as, for example, epoxides) and to avoid the unnecessary production of enantiomers or diastereoisomers of metabolites and conjugates, without losing pharmacological activity. See Benes et al., "Anticonvulsant and Sodium Channel-Blocking Properties of Novel 10,11-Dihydro-5H-dibenz[b,f]azepine-5-carboxamide Derivatives," J. Med. Chem., 42, 2582-2587 (1999).

Like carbamazepine and oxcarbazepine, eslicarbazepine acetate is believed to be a voltage-gated sodium channel (VGSC) blocker that competitively interacts with site 2 of the inactivated state of the sodium channel. The affinity for this state of the channel is similar to that of carbamazepine, while the affinity for the resting state of the channel is about 3-fold lower than that of carbamazepine. This profile may suggest an enhanced inhibitory selectivity of eslicarbazepine acetate for rapidly firing neurons over those displaying normal activity. See Bonifacio et al., "Interaction of the Novel Anticonvulsant, BIA 2-093, with Voltage-Gated Sodium Channels: Comparison with Carbamazepine," Epilepsia, 42, 600-608 (2001).

Evaluation of the metabolic profile of eslicarbazepine acetate, following chiral analysis, in liver microsomes from rats, dogs, monkeys and humans was found to give the S(+) enantiomer of licarbazepine, (S)-(+)-10,11-dihydro-10-hydroxy-5H dibenz/b,f/azepine-5-carboxamide (also known as "eslicarbazepine"), and not the R(-) form of licarbazepine, (R)-(-)-10,11-dihydro-10-hydroxy-5H dibenz/b,f/azepine-5-carboxamide (also known as "R-licarbazepine").

Studies in humans have shown that, after oral administration, eslicarbazepine acetate appears to be rapidly and extensively metabolized to the active metabolite eslicarbazepine and, in a minor extent, to R-licarbazepine. See Silveira et al., "BIA 2-093 Pharmacokinetics in Healthy Elderly Subjects," Epilepsia, 45 (suppl. 3), 157 (2004). For example, the plasma concentrations of the parent drug (eslicarbazepine acetate) have been systematically found below the limit of quantification (LOQ) of the assay (10 ng/mL). *See Almeida I*; Almeida, L. & Soares-da-Silva, P., "Safety, Tolerability and Pharmacokinetic Profile of BIA 2-093, a Novel Putative Antiepileptic Agent, during First Administration to Humans," Drugs R&D, 4, 269-284 (2003) (herein referred to as "*Almeida II*"). When a non-chiral method is used, the assay does not distinguish between eslicarbazepine and the R-enantiomer, and the mixture was reported as "BIA 2-005" or "racemic licarbazepine."

The inventors performed entry-into-man studies in healthy subjects, the results of which they described in the *Almeida I* and *Almeida II* articles. In these studies, the healthy subjects received a single oral dose of eslicarbazepine acetate wherein the dose ranged from 20 mg to 1200 mg (*see Almeida II*), and multiple daily-doses of eslicarbazepine acetate ranging from 200 mg twice-daily to 1200 mg once-daily (*see Almeida I*). Further studies (not yet published) by the inventors have investigated higher doses of eslicarbazepine acetate, including, for example, doses ranging up to 2400 mg once-daily. The studies showed that BIA 2-005 maximum observed plasma concentration (Cₘₐₓ) was attained at about 1 hour to about 4 hours post-dose (tₘₐₓ), the extent of systemic exposure to BIA 2-005 was approximately dose-proportional, and steady-state of BIA 2-005 plasma concentrations was attained at about 4 to 5 days. The mean renal clearance of BIA 2-005 from plasma was about 20-30 mL/min, and the total amount of BIA 2-005 recovered in the urine was approximately 20% and 40% within 12 hours and 24 hours post-dose, respectively.

The studies also showed that the apparent terminal half-life of BIA 2-005 ranged from about 8 hours to about 17 hours. *See*, *e.g*., *Almeida II.*

U.S. Patent No. 6,296,873 discloses a sustained release delivery system for carbamazepine, which has a half-life ranging from 25 hours to 85 hours. To avoid adverse effects, U.S. Patent No. 6,296,873 teaches that the carbamazepine should be administered in tablet form up to two or more times daily to slowly release the compound to maintain concentration levels between 4-12 µg/mL. Such a delivery system requires a form that is capable of delivering the compound over an extended period of time, such as a tablet form.

WO 03/042182 relates to the use of licarbazepine for the treatment of affective and attention disorders, neuropathic pain and neuropathic pain related disorders.

D. Hainzl et al. Epilepsy Research, 2001, Vol. 44, No.2/03, 197-206 discloses a study to evaluate the metabolism of eslicarbazepine acetate and another antiepileptic agent, BIA-2 059.

E. Carrazana et al., Journal of Pain and Symptom Management, 2003, Vol. 25, No. 5, S31-S35 relates to the rationale and evidence for the use of oxcarbazepine in the treatment of neuropathic pain.

EP 1477480A discloses a method for the racemisation of (S)-(+)- and (R)-(-)-10,11-dihydro-10-hydroxy-5H dibenz/b,f/azepine-5-carboxamide and optically enriched mixtures thereof.

US 2004/038874 relates to a method for the treatment of persistent pain by inhibiting inflammation using, for example a TNF-alpha inhibitor.

WO 2004/071513 relates to the use of a mixture of enantiomers of licarbazepine for the treatment of neuropathic pain.

In one aspect of the present disclosure, the inventors have unexpectedly discovered an enhanced efficacy of eslicarbazepine acetate in the treatment of various conditions using once-daily administration compared to twice-daily administration. This discovery is particularly surprising because the apparent half-life of eslicarbazepine acetate (t_{1/2} = about 8 hours to about 17 hours) is significantly shorter than the half-life of carbamazepine (t_{1/2} = 25 hours to 85 hours), a compound typically administered 3-4 times daily.

In another aspect of the present disclosure, the inventors have also unexpectedly discovered an enhanced exposure to eslicarbazepine after once-daily administration of eslicarbazepine acetate versus the twice-daily regimen in humans. Once-daily administration of eslicarbazepine acetate surprisingly provides an increase of exposure to eslicarbazepine than the same drug dosage divided into twice-daily doses.

### BRIEF DESCRIPTION OF THE DRAWINGS

**Figure 1**: Percentage reduction in seizure number in each dose period versus baseline (400 mg once-daily versus twice-daily and placebo; 800 mg once-daily versus twice-daily and placebo; 1200 mg once-daily versus twice-daily and placebo).
**Figure 2****:** Mean (95% Cl) trough plasma concentrations (µg/mL) of BIA 2-005 following a daily dose of 400 mg, 800 mg and 1200 mg of BIA-2-093 administered once-daily (o.d.) or twice-daily (b.i.d.).

### DETAILED DESCRIPTION

The foregoing and following aspects and embodiments, including the studies discussed herein, are described and illustrated in a manner intending to be exemplary only, and should not be construed as limiting in scope.

One aspect of the present disclosure relates to a method for treating post-herpetic neuralgia in a patient in need thereof by administering a pharmaceutical composition comprising eslicarbazepine acetate in a pharmacologically effective amount.

In one exemplary embodiment of the present disclosure, the pharmaceutical composition comprising eslicarbazepine acetate is administered in a once-daily dosing regimen.

In another exemplary embodiment of the present disclosure, the pharmaceutical composition is administered in a dosage intended to maximize the total exposure to eslicarbazepine, as measured by the rate of exposure and extent of exposure (Cₘₐₓ and AUC_{o-τ}).

In an exemplary embodiment of the present disclosure, the disease or condition treated is post-herpetic neuralgia.

Another aspect of the present disclosure relates to a pharmaceutical composition comprising eslicarbazepine acetate and at least one pharmaceutical excipient, at least one auxiliary substance, at least one carrier material, or combinations thereof for treating post-herpetic neuralgia.

A further aspect of the present disclosure relates to a method of preparing a pharmaceutical composition for treating post-herpetic neuralgia comprising combining eslicarbazepine acetate with at least one excipient, at least one auxiliary substance, at least one carrier material, or combinations thereof. Suitable excipients, carrier materials, and other auxiliary substances which would be useful in the present invention are known to those skilled in the art, and would be readily determined. Methods for preparing pharmaceutical compositions are also known to those skilled in the art.

In one exemplary embodiment of the present disclosure, the pharmaceutical composition may be in tablet form and may comprise at least one excipient, auxiliary substance, and/or carrier material. The at least one excipient, auxiliary substance, and/or carrier material may be chosen from, for example, povidone, croscarmellose sodium, magnesium stearate, saccharin sodium, dibasic calcium phosphate dihydrate, sodium lauryl sulphate, flavorings, and combinations thereof. Exemplary tablets may be formed using granulation liquids, such as, for example, purified water and ethanol.

In another exemplary embodiment of the present disclosure, the pharmaceutical composition may be in oral suspension form and may comprise at least one excipient, auxiliary substance, and/or carrier material. The at least one excipient, auxiliary substance, and/or carrier material may be chosen from, for example, xantham gum, macrogol stearate (such as, for example, Myrj 59 P, produced by UNIQEMA), methylparaben, propylparaben, saccharin sodium, sorbitol, buffers, flavorings, and combinations thereof.

In a further exemplary embodiment of the present disclosure, the active ingredient of the pharmaceutical composition may consist essentially of eslicarbazepine acetate.

In a further aspect of the present disclosure, eslicarbazepine acetate may be administered to a patient in an amount resulting in a maximum plasma concentration (Cₘₐₓ) of eslicarbzepine greater than about 7,400 ng/mL. In other exemplary embodiments, eslicarbazepine acetate may be administered to a patient in an amount resulting in a Cₘₐₓ of eslicarbazepine greater than about 12,000 ng/mL or greater than about 16,100 ng/mL. In further exemplary embodiments, eslicarbazepine acetate may be administered to a patient in an amount resulting in a Cₘₐₓ of eslicarbazepine greater than about 22,700 ng/mL, such as greater than about 36,500 ng/ml, greater than about 45,200 ng/mL, or more.

In a further exemplary embodiment, eslicarbazepine acetate may be administered to a patient in an amount resulting in a maximum plasma concentration (Cₘₐₓ) of eslicarbzepine up to about 58,800 ng/mL or up to about 67,800 ng/mL. In a further exemplary embodiment, eslicarbazepine acetate may be administered to a patient in an amount resulting in a maximum plasma concentration (Cₘₐₓ) of eslicarbzepine up to about 885,000 ng/mL or up to about 1,000,000 ng/mL.

For example, a once-daily dose of about 400 mg may be administered to a patient resulting in a maximum plasma concentration (Cₘₐₓ) of eslicarbazepine greater than about 7,400 ng/mL. As a further example, a once-daily dose of about 800 mg or about 1200 mg may be administered to a patient resulting in a Cₘₐₓ of eslicarbazepine greater than about 16,100 ng/mL or greater than about 22,700 ng/mL, respectively. In other examples, eslicarbazepine acetate may be administered in a once-daily dose greater than about 1200 mg, such as about 1800 mg or about 2400 mg, to result in a Cₘₐₓ of eslicarbazepine greater than about 36,500 ng/mL, about 45,200 ng/mL, respectively.

In a further aspect of the present disclosure, eslicarbazepine acetate may be administered to a patient in an amount resulting in an area under the concentration curve (which corresponds to the extent of systemic exposure) over the dosing interval (AUC_{0-τ}) of eslicarbazepine greater than about 111,000 ng·h/mL. In other exemplary embodiments, eslicarbazepine acetate may be administered to a patient in an amount resulting in an AUC_{0-τ} of eslicarbazepine greater than about 240,000 ng·h/mL or greater than about 375,000 ng·h/mL, respectively. In other examples, eslicarbazepine acetate may be administered to a patient in an amount resulting in an AUC_{0-τ} of eslicarbazepine greater than about 595,000 ng·h/mL, greater than about 790,000 ng·h/mL, or more.

For example, a once-daily dose of about 400 mg may be administered resulting in an area under the concentration curve (which corresponds to the extent of systemic exposure) over the dosing interval (AUC_{0-τ}) of eslicarbazepine greater than about 111,000 ng·h/mL. In other exemplary embodiments, a once-daily dose of about 800 mg or about 1200 mg may be administered resulting in an AUC_{0-τ} of eslicarbazepine greater than about 240,000 ng·h/mL or greater than about 375,000 ng·h/mL, respectively. In other examples, eslicarbazepine acetate may be administered in a once-daily dose greater than about 1200 mg, such as about 1800 mg, about 2400 mg, or more, to result in a respective AUC_{0-τ} of eslicarbazepine greater than about 595,000 ng·h/mL, greater than about 790,000 ng·h/mL, or more.

In one exemplary embodiment of the present disclosure, a once-daily dose may be administered in a dosage comprising at least about 400 mg of eslicarbazepine acetate. In another exemplary embodiment, a once-daily dose may be administered in a dosage comprising an amount of eslicarbazepine acetate ranging from about 800 mg to about 1200 mg. In further exemplary embodiments, a once-daily dose may be administered in a dosage comprising an amount of eslicarbazepine greater than about 1200 mg, such as about 1800 mg, about 2400 mg, or more.

The pharmaceutical composition comprising eslicarbazepine acetate may optionally be administered by any route known to those skilled in the art, and may be in a form chosen from, for example, tablets or oral suspensions, or other forms.

According to another aspect of the invention there is provided a pharmaceutical unit dosage composition comprising about 400 to 1200 mg of eslicarbazepine acetate. said unit dosage form being suitable for oral administration for the treatment of the diseases mentioned above, excluding epilepsy, up to a maximum dose of 1200 mg per day.

The term "about" as used herein is meant to signify that the number modified by the term may be considered an approximation that may vary depending upon the desired properties or effect sought by the particular application, and thus should be considered to encompass the range that one of skill in the art would understand to achieve the desired or recited properties or effect.

A "method of treating" as described herein refers to administering to a patient the compound described in any amount effective to reduce the effects of, counteract, or eliminate the disease or condition being treated, or the symptoms thereof.

A "method for increasing the exposure to eslicarbazepine in a patient" as described herein refers to administering to a patient the compound described in any amount effective to increase the plasma concentration of eslicarbazepine in the patient over the dosage interval. This may, for example, be an increase due to once-daily dosing relative to twice-daily dosing.

A "pharmacologically effective amount" of eslicarbazepine acetate in a pharmaceutical composition as described herein refers to any amount sufficient to have the desired pharmacological activity.

All effective amounts as described herein will vary according to various well-known and understood factors, such as, for example, the condition being treated and the physiological characteristics of the patient being treated. Accordingly, the effective amount will be well within the ability of one skilled in the art to determine.

### STUDY MATERIALS AND METHODS

The following demonstrates, as one example of the present disclosure, the determination and administration of an effective amount of a pharmaceutical composition comprising eslicarbazepine acetate to treat epilepsy in patients in need thereof. The effective amount of a pharmaceutical composition to treat other diseases and/or conditions would be determinable by one skilled in the art based on the techniques and concepts disclosed herein and known in the art.

The effects of eslicarbazepine acetate in humans was studied in at least the following clinical studies. In the first study, a placebo-controlled therapeutic exploratory study, once-daily and twice-daily dosing was compared in epileptic patients refractory to standard anti-epileptic drug therapy. In the second study healthy subjects received either a once-daily (o.d.) oral dose of 900 mg of eslicarbazepine acetate or a twice-daily (b.i.d.) dose of 450 mg of eslicarbazepine acetate. In the third study, healthy subjects received single oral doses of eslicarbazepine acetate ranging from 20 mg to 2400 mg, and repeated once-daily (o.d.) oral doses ranging from 400 mg to 2400 mg of eslicarbazepine acetate.

The bioequivalence of tablets and oral suspensions was proven in a relative bioavailability study.

### Study in Epileptic Patients

This clinical trial was a double-blind, randomized, placebo-controlled study performed by 20 centers in Croatia, Czech Republic, Germany, Lithuania and Poland. The stated objectives of the study were to assess the efficacy and safety of BIA 2-093 as adjunctive therapy in patients with refractory partial epilepsy. In total, 143 patients aged 18-65 years with at least 4 partial-onset seizures per month in spite of treatment with 1 or 2 antiepileptic drugs (AEDs) (e.g., phenytoin, valproate, primidone, phenobarbital, lamotrigine, gabapentin, topiramate or clonazepam) were randomly assigned to one of three groups: treatment with placebo (n=47), BIA 2-093 once-daily (n=50), or BIA 2-093 twice-daily (n=46), during 12 weeks (plus 1 week of tapering off). For the first 4 weeks, daily dose was 400 mg. Then, daily doses were increased to 800 mg (weeks 5-8), and finally to 1200 mg (weeks 9-12). Tablets with strengths of 200 mg, 400 mg, and 600 mg eslicarbazepine acetate and placebo tablets were manufactured by BIAL (S. Mamede do Coronado, Portugal) in accordance with Good Manufacturing Practice. The assay plasma to determine the concentration of BIA 2-005 was performed with a non-chiral method using isocratic liquid chromatography (LC) with single quadrupole mass spectrometric detection (MS), as described herein. *See*, *e.g., Almeida I* and *Almeida II*.

### Study in Healthy Human Volunteers

### Trial A

This human pharmacology trial was a study to investigate the steady-state pharmacokinetics of once-daily and twice-daily regimens of eslicarbazepine acetate in healthy subjects. The study was a single center, open-label, randomized, two-way crossover study in 12 healthy volunteers (6 males and 6 females) that consisted of two 8-day treatment periods separated by a washout period of 10-15 days. On each of the treatment periods the volunteers received either a daily oral dose of eslicarbazepine acetate 900 mg once-daily (o.d.) or eslicarbazepine acetate 450 mg twice-daily (b.i.d.). Tablets with a strength of 450 mg of eslicarbazepine acetate, manufactured by BIAL (S. Mamede do Coronado, Portugal) in accordance with Good Manufacturing Practice, were used.

Blood samples for drug plasma assays were taken at the following times:
*Phase A:*
   pre-dose, and ½, 1, 1½, 2, 3, 4, 6, 8, 12, 24, 36, 48, 72, and 96 hours post-dose;
*Phase B:*
   day 5 to day 11 (inclusive): before the daily dose (for "trough" concentrations assay);
   day 12: pre-dose, and ½, 1, 1½, 2, 3, 4, 6, 8, 12, 24, 36, 48, 72, 96, and 120 hours post-dose.
Blood samples were drawn either by direct venipuncture or via an intravenous catheter into lithium heparin tubes and centrifuged at approximately 1500 g for 10 minutes at 4°C. The resulting plasma was separated into 2 equal aliquots of 1 mL and stored at -20°C until required for analysis.

Plasma concentrations of eslicarbazepine acetate, eslicarbazepine, and R-licarbazepine were determined using isocratic liquid chromatography (LC) with single quadrupole mass spectrometric detection (MS).

The method involved the addition of 500 µL of approximately 0.5 µg/mL of 10,11-dihydrocarbamazepine (internal standard prepared in acetonitrile:water, 3:97, v:v) to 250 µL of plasma (centrifuged at 1800 rpm, prior to analysis) in a polypropylene tube. After vortex mixing for 10 seconds, the mixture was transferred to a Schleicher and Schuell C18/100 mg 96 well solid phase extraction plate. Each well was preconditioned with 800 µL methanol, followed by 800 µL acetonitrile and 800 µL acetonitrile:water (3:97, v:v) prior to application of the total sample volume. Each polypropylene tube was then washed with 500 µL acetonitrile:water (3:97, v:v) and the washings transferred to the respective well. The compounds were eluted into a collection plate with 750 µL acetonitrile and the extract evaporated to dryness under oxygen-free nitrogen, at 40°C. All solid phase extraction manipulations were undertaken using the Tomtec QUADRA 96^{®} Model 320 system and a vacuum was applied at each elution step. The final extract was reconstituted in 100 µL of water:methanol (90:10, v:v) and mixed. The collection plate was then centrifuged at approximately 3000 rpm (at approximately 4°C, for approximately 10 minutes) prior to analysis. An aliquot of the final extract (10 µL) was injected onto the LC-MS system.

The LC-MS system used in the analysis consisted of a Perkin Elmer series 200 micro pump, a Perkin Elmer series 200 autosampler, and a Perkin Elmer/Sciex API 150EX single quadrupole mass spectrometer fitted with a Turbo IonSpray^{®} source. Separation was achieved using a LichroCART 250-4 ChiraDex column (β-cyclodextrin, 5 µm), a LichroCART 4-4 ChiraDex column guard column (β-cyclodextrin, 5 µm), a Jones Chromatography 7971 column heater at 50°C, a mobile phase A (0.2 mM sodium acetate, aq) and a mobile phase B (0.2 mM sodium acetate, MeOH). The MS detector was operated in positive ion mode with mass transitions for BIA 2-093, eslicarbazepine, R-licarbazepine, and the internal standard of 319.16 amu (200 ms), 277.08 amu (200 ms), 277.08 amu (200 ms) and 261.05 amu (200 ms), respectively. The limit of quantification of the assay was 10 ng/mL for eslicarbazepine acetate and 100 ng/mL for eslicarbazepine and R-licarbazepine.

Eslicarbazepine acetate, (S)-(-)-10-acetoxy-10,11-dihydro-5H-dibenz/b,f/azepine-5-carboxamide; eslicarbazepine, (S)-(+)-10,11-dihydro-10-hydroxy-5H dibenz/b,f/azepine-5-carboxamide; and R-licarbazepine, (R)-(-)-10,11-dihydro-10-hydroxy-5H dibenz/b,f/azepine-5-carboxamide, were synthesized in the Laboratory of Chemistry, BIAL, with purities >99.5%. The internal standard, 10,11-dihydrocarbamazepine was supplied by Sigma-Aldrich (St. Louis, MO).

The pharmacokinetic parameters were derived from non-compartmental analysis using WinNonlin (Version 4.0, Pharsight Corporation, Mountain View, California). The following parameters were derived, where appropriate, from the individual plasma concentration-time profiles: maximum observed plasma concentration (Cₘₐₓ); time of occurrence of Cₘₐₓ (tₘₐₓ); area under the plasma concentration versus time curve (AUC) from time zero to the last sampling time (t) at which concentrations were at or above the limit of quantification (AUC₀₋ₜ), calculated by the linear trapezoidal rule; AUC over the dosing interval (AUC_{τ}), i.e., 24 hours and 12 hours in the once-daily and the twice-daily group, respectively; AUC from time zero to infinity (AUC_{0-∞}), calculated from AUC₀₋ₜ + (Cₗₐₛₜ/λ_{z}), where Cₗₐₛₜ is the last quantifiable concentration; apparent terminal rate constant (λ_{z}) calculated by log-linear regression of the terminal segment of the plasma concentration versus time curve; apparent terminal half-life (t_{½}), calculated from In 2/λ_{z}.

Actual sampling times were used for the pharmacokinetic analysis. Where an AUC was extrapolated to infinity, the percentage of the extrapolated area to the total area was assessed; if greater than 20%, the AUC value was flagged as unreliable. Plasma concentrations below the limit of quantification of the assay (BLQ) were taken as zero for all calculations. All calculations were made using raw data. Values for tₘₐₓ were displayed as nominal times.

Summary statistics for each group and schedule sampling time were reported, as appropriate, using the geometric mean, arithmetic mean, standard deviation (SD), coefficient of variation (CV), median, minimum, and maximum. Comparisons between elderly versus young groups for the single-dose and multiple-dose data were based on analysis of variance (one-way ANOVA) of the logarithmic transformed Cₘₐₓ, AUC_{τ} and AUC_{0-∞} parameters. A tₘₐₓ comparison between age groups was performed assuming a non-parametric approach using the Wilcoxon signed rank test. In addition, differences in logarithmic transformed parameters (Cₘₐₓ, AUC_{τ} and AUC_{0-∞}) and their associated 95% confidence intervals (95%Cl) were estimated between age groups to take the form of ratios on a linear scale. The median values and differences of tₘₐₓ between age groups and 95%Cl were reported. All tests of significance were performed at the p=0.05 level. The statistical package SAS (Version 8.2, SAS Institute Inc, Cary, NC) was used.

### Trial B

This human pharmacology trial was a study to determine the pharmacokinetics of eslicarbazepine acetate following single and repeated doses. The study integrated the results of three double-blind, randomized, placebo-controlled trials. To measure the pharmacokinetics of eslicarbazepine acetate following single doses, oral single doses of eslicarbazepine acetate ranging from 20 mg to 2400 mg were administered to healthy young male subjects (6 subjects per dose). The pharmacokinetics of eslicarbazepine acetate following repeated doses were measured by administering repeated oral doses ranging from 400 mg to 2400 mg of eslicarbazepine acetate to healthy young male subjects (6 subjects per dose) over a period of 8 days. Analytical test methods and experimental procedures were similar to those described for *Trial A* above.

### STUDY RESULTS

### Study in Epileptic Patients

### Baseline Characteristics

At baseline, the treatment groups were homogenous with regard to age, height, weight, and body mass index. All 143 patients were Caucasian. With respect to gender, there were relatively more female patients in the twice-daily group than in the once-daily and placebo groups (65.2%, 56.0% and 57.4%, respectively); this difference did not significantly affect the results. No significant differences were found in the number of AEDs used: respectively 30.0%, 34.8% and 29.8% of patients in the once-daily, twice-daily, and placebo groups were treated with 1 AED; the remaining patients were treated with 2 AEDs. The most frequently used concomitant AEDs were valproic acid (68.0%, 60.9% and 66.0% of patients in the once-daily, twice-daily, and placebo groups, respectively), topiramate (36.0%, 34.8%, and 21.3%, respectively) and lamotrigine (30.0%, 28.3%, and 31.9%, respectively).

At baseline, mean duration of epilepsy was 16.7, 19.5, and 20.0 years in the once-daily, twice-daily, and placebo groups, respectively. With respect to seizure type frequency, IA simple partial, IB complex partial, and IC partial evolving to secondarily generalized were present in, respectively, 34.0%, 72.0%, and 80.0%, in the once-daily group; 37.0%, 71.7%, and 80.4%, in the twice-daily group; and 27.7%, 80.9%, and 72.3%, in the placebo group. The mean of total number of seizures per month prior to the study was 14.1, 13.6, and 11.8, in the once-daily, twice-daily, and placebo groups, respectively.

### Efficacy Results

The proportion of patients with a 50% or greater reduction in seizure frequency in the treatment period compared to the baseline period in the intention-to-treat (ITT) population (n=143) was the primary efficacy endpoint. At the dose of 1200 mg/day (weeks 9-12), the proportion of responders in the once-daily group (54%) was significantly higher (p=0.008) than in the placebo group (28%). The proportion of responders in the once-daily group (54%) was also higher than in the twice-daily (41 %) group. At the dose of 800 mg/day (weeks 5-8), the proportion of responders in the once-daily group (58%) was significantly higher (p<0.05) than in the twice-daily (33%) and placebo (38%) groups. At this dose level, no significant difference was found between the twice-daily and the placebo groups.

Secondary endpoints include the reduction in total seizure frequency, proportion of seizure-free patients, distribution of responders, comparison of once and twice daily regimens, and investigator's and patient's global evaluation.

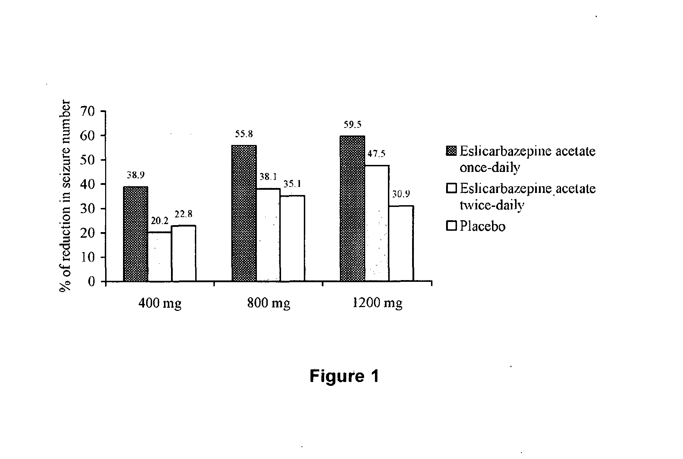

The greatest decrease in the number of seizures was achieved with 1200 mg and 800 mg once-daily doses, and the results with the once-daily group were better than those obtained in the twice-daily group (Figure 1). For all dosages (400 mg, 800 mg, and 1200 mg), patients receiving once-daily doses of eslicarbazepine acetate had a substantially greater reduction in the number of seizures compared to patients in the twice-daily and placebo groups.

The number of seizures in patients receiving 1200 mg and 800 mg once-daily doses of eslicarbazepine acetate was reduced by 59.5% and 55.8%, respectively. In comparison, seizures in patients receiving 1200 mg and 800 mg twice-daily doses reduced by 47.5% and 38.1%, respectively. Patients receiving a 400 mg once-daily dose of eslicarbazepine acetate experienced a 38.9% reduction in the number of seizures, almost twice the reduction in seizures observed in patients receiving 400 mg twice-daily doses of eslicarbazepine acetate (20.2%).

At the end of the 12-week treatment phase, 27.9% of the patients in the once-daily dosing group became seizure-free.

In addition, the assessment of efficacy by the investigator (CGI - Clinical Global Impression) and of acceptability by patient was rated best in the once-daily group.

### Pharmacokinetic Results

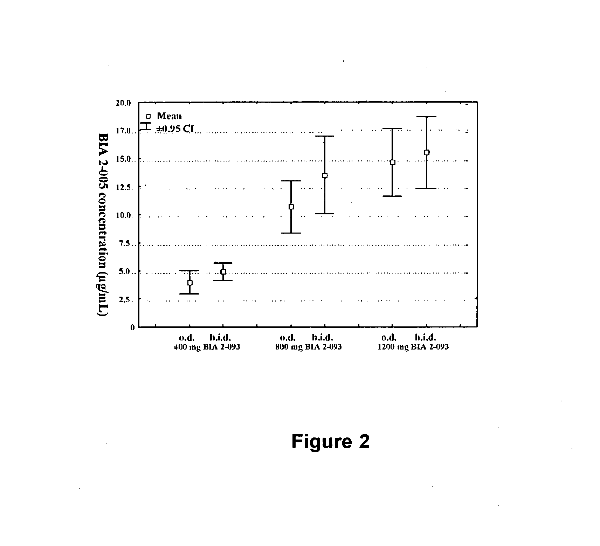

Plasma/serum samples for the "trough" (pre-dose) levels of BIA 2-005 and concomitant AEDs were collected at all visits but V5 (post-study visit). The objective was to characterize the influence of eslicarbazepine acetate on the pharmacokinetic behavior of the concomitant AEDs (e.g., phenytoin, valproate, primidone, phenobarbital, lamotrigine, gabapentin, topiramate, and clonazepam). The mean trough plasma concentrations of BIA 2-005 are displayed in Table 1. As shown in Figure 2, no significant differences in the BIA 2-005 trough (pre-dose) values between the once-daily and twice-daily groups were found.

**Table 1: Trough plasma concentrations of BIA 2-005 following oral administration of eslicarbazepine acetate once-daily (o.d.) and twice-daily (b.i.d)**

| | 400 mg/day | | 800 mg/day | | 1200 mg/day | |
|---|---|---|---|---|---|---|
| | o.d. group | b.i.d. group | o.d. group | b.i.d. group | o.d. group | b.i.d. group |
| Mean (µg/mL) | 4.0 (3.2) | 4.9 (2.3) | 10.7 (7.0) | 13.5 (9.6) | 14.6 (8.8) | 15.5 (8.8) |

Results expressed as arithmetic means with the correspondent standard deviations (sd) in parenthesis.

The relatively small number of patients being administered phenytoin, primidone, phenobarbital, gabapentin, and clonazepam precluded the appropriate characterization of the eventual effect of eslicarbazepine acetate on the pharmacokinetic behavior of these concomitant AEDs. For valproate, lamotrigine and topiramate, the number of patients was also small, but an exploratory analysis of the effect of eslicarbazepine acetate on the trough blood values of these concomitant AEDs was performed. The mean trough serum concentrations of valproate were not significantly changed by concomitant administration of eslicarbazepine acetate once-daily (7.0%; 95% IC: -7.6, 36.2) or twice-daily (6.3%; 95% IC: -7.5, 20.1). In the placebo group, a significant increase in the serum levels of valproate was noticed (25.4%; 95% IC: 5.1, 45.8). With respect to lamotrigine, its serum levels were not significantly changed when eslicarbazepine acetate once-daily (-10.0%; 95% IC: -46.2, 26.2) or placebo (12.6%; 95% IC: -12.6, 37.8) were added to therapy. With eslicarbazepine acetate twice-daily, the serum levels of lamotrigine decreased significantly (-46.7%; 95% IC: -69.7; -23.8). With respect to topiramate, its serum levels were not significantly changed when eslicarbazepine acetate once-daily (-15.2%; 95% IC: -34.8, 4.4) was added to the therapy. With eslicarbazepine acetate twice-daily, the serum levels of topiramate decreased significantly (-32.4%; 95% IC: -49.5; -15.3). One skilled in the art will know whether a change in serum levels is significant.

### Study in Healthy Human Volunteers

### Trial A

### Pharmacokinetic Results

Eslicarbazepine acetate was shown to be extensively metabolized to eslicarbazepine and, in a minor extent, to R-licarbazepine. The steady-state of eslicarbazepine plasma concentrations was attained at 4 to 5 days of administration in both groups.

Following the last dose, in the once-daily group, mean Cₘₐₓ of eslicarbazepine and R-licarbazepine was, respectively, 22,210 ng/mL and 674 ng/mL and occurred at (median tₘₐₓ) 2.45 hours and 9.42 hours post-dose, respectively. Mean AUC₀₋ₜ of eslicarbazepine and R-licarbazepine was 381,601 ng·h/mL and 19,600 ng·h/mL, respectively. In the twice-daily group, mean Cₘₐₓ of eslicarbazepine and R-licarbazepine was 16,667 ng/mL and 718 ng/mL, respectively, and occurred (median tₘₐₓ) at 2.09 hours and 6.40 hours post-dose, respectively. Mean AUC₀₋ₜ of eslicarbazepine and R-licarbazepine was 283,014 ng·h/mL and 19,661 ng·h/mL, respectively. Following multiple administration of eslicarbazepine acetate for 8 days, eslicarbazepine was shown to be the major metabolite, representing approximately 95% and 96% of total systemic drug exposure (as assessed by AUC₀₋₂₄) in once-daily and twice-daily subjects, respectively. Tables 2 and 3 depict the eslicarbazepine and R-licarbazepine pharmacokinetic parameters in the once-daily and twice-daily groups following the last dose of eslicarbazepine acetate. The total exposure of healthy volunteers to eslicarbazepine in the once-daily group was unexpectedly at least 26% higher than in the twice-daily group.

**Table 2: Mean pharmacokinetic parameters of eslicarbazepine and R-licarbazepine following a multiple oral dose of 900 mg eslicarbazepine acetate once-daily.**

| | Cₘₐₓ (ng/mL) | tₘₐₓ (h) | AUC₀₋ₜ (ng·h/mL) | AUC_{0-τ} (ng·h/mL) | AUC_{0-∞} (ng·h/mL) | t_{1/2} (h) |
|---|---|---|---|---|---|---|
| Eslicarbazepine | | | | | | |
| n | 11 | 11 | 11 | 11 | 11 | 11 |
| Aₘₑₐₙ | 22210 | 2.45 | 381601 | 294019 | 389344 | 9.12 |
| SD | 7257 | 0.879 | 95368 | 58364 | 97383 | 1.19 |

| R-licarbazepine | | | | | | |
|---|---|---|---|---|---|---|
| n | 12 | 12 | 12 | 12 | 12 | 12 |
| Aₘₑₐₙ | 674 | 9.42 | 19600 | 13397 | 23989 | 15.0 |
| SD | 184 | 6.48 | 6763 | 3187 | 7144 | 3.41 |

| | | | | | | |
|---|---|---|---|---|---|---|
| n = Number of subjects; Aₘₑₐₙ = Arithmetic mean; SD = Standard deviation. | | | | | | |

**Table 3: Mean pharmacokinetic parameters of eslicarbazepine and R-licarbazepine following a multiple oral dose of 900 mg eslicarbazepine acetate twice-daily.**

| | Cₘₐₓ (ng/mL) | tₘₐₓ (h) | AUC₀₋ₜ (ng·h/mL) | AUC_{0-τ} (ng·h/mL) | AUC_{0-∞} (ng·h/mL) | t_{1/2} (h) |
|---|---|---|---|---|---|---|
| Eslicarbazepine | | | | | | |
| n | 11 | 11 | 11 | 11 | 11 | 11 |
| Aₘₑₐₙ | 16667 | 2.09 | 283014 | 142080 | 289792 | 9.17 |
| SD | 3981 | 0.664 | 74203 | 25933 | 74346 | 1.49 |

| R-licarbazepine | | | | | | |
|---|---|---|---|---|---|---|
| n | 10 | 10 | 10 | 10 | 10 | 10 |
| Aₘₑₐₙ | 718 | 6.40 | 19661 | 7783 | 23807 | 14.8 |
| S D | 184 | 3.06 | 6049 | 2083 | 7150 | 4.09 |

| | | | | | | |
|---|---|---|---|---|---|---|
| n = Number of subjects; Aₘₑₐₙ = Arithmetic mean; SD = Standard deviation. | | | | | | |

### Trial B

### Pharmacokinetic Results

As in *Trial A*, eslicarbazepine acetate was extensively metabolized to eslicarbazepine and, in a minor extent, to R-licarbazepine. The steady-state of eslicarbazepine plasma concentrations was attained at 4 to 5 days of once-daily dosing.

Following the last dose, in the repeated once daily group, mean Cₘₐₓ of eslicarbazepine ranged from 8,800 ng/ML·(16.0% coefficient of variation, CV) for 400 mg doses of eslicarbazepine acetate to 56,500 ng/ML·(20.0% CV) for 2400 mg doses of eslicarbazepine acetate. The maximum plasma concentration for all dosages occurred (median tₘₐₓ) at 2 hours to 3.5 hours. The mean area under the concentration for the dosing interval of 24 hours, AUC₀₋₂₄ₕ, ranged from 126,300 ng/ML for 400 mg once-daily doses of eslicarbazepine acetate to 905,900 ng/ML for 2400 mg once-daily doses of eslicarbazepine acetate. Tables 4 and 5 depict the eslicarbazepine and R-licarbazepine pharmacokinetic parameters following the single dose of eslicarbazepine acetate and the pharmacokinetic parameters following the last of the repeated doses of eslicarbazepine acetate.

**Table 4: Mean pharmacokinetic parameters of eslicarbazepine and R-licarbazepine following single doses of eslicarbazepine acetate (n=6 subjects per dose group).**

| Dose | **Mean Cₘₐₓ** ng/mL (%CV) | **Median tₘₐₓ** h (range) | **Mean AUC₀₋₂₄ₕ** ng·h/mL (%CV) | **Mean apparent t**_{½} h (%CV) |
|---|---|---|---|---|
| 20 mg | 300 (18.7) | 0.8 (0.5-0.8) | 2,400 (16.2) | 9.1 (15.9) |
| 50 mg | 900 (24.7) | 0.8 (0.5-2) | 6,700 (12.7) | 8.1 (9.1) |
| 100 mg | 1,500 (13.8) | 1.5 (0.5-2) | 16,400 (11.7) | 9.3 (8.7) |
| 200 mg | 2,900 (16.2) | 1.5 (0.8-2.5) | 30,500 (23.7) | 8.4 (18.8) |
| 400 mg | 5,200 (11.6) | 4 (4-5) | 81,500 (10.8) | 11.7 (18.6) |
| 600 mg | 8,500 (20.0) | 4 (0.5-5) | 119,700 (17.4) | 12.3 (14.8) |
| 900 mg | 15,000 (18.2) | 2.3 (0.8-4) | 210,300 (10.6) | 16.3 (31.9) |
| 1200 mg | 18,600 (16.3) | 4 (2-6) | 285, 700 (16.7) | 16.5 (6.8) |
| 1800 mg | 34,600 (16.3) | 3.5 (3-6) | 507,600 (17.0) | 11.8 (11.7) |
| 2400 mg | 35,900 (42.6) | 3 (1.5-6) | 445.6 (26.1) | 11.1 (21.1) |

| | | | | |
|---|---|---|---|---|
| CV = Coefficient of variation (%); Cₘₐₓ = Maximum plasma concentration; AUC₀₋₂₄ₕ = Area under the curve of plasma concentration-time over 24 h; tₘₐₓ = Time to Cₘₐₓ; t_{1/2} = Elimination half-life | | | | |

**Table 5: Mean pharmacokinetic parameters of eslicarbazepine and R-licarbazepine following the last dose of an 8-day repeated dose regimen of eslicarbazepine acetate (n=6 subjects per dose group).**

| **Dose** | **Mean Cₘₐₓ** ng/mL (%CV) | **Median tₘₐₓ** h (range) | **Mean AUC₀₋₂₄ₕ** ng·h/mL (%CV) | **Mean apparent t_{½}** h (%CV) |
|---|---|---|---|---|
| 400 mg o.d. | 8,800 (16.0) | 3 (0.5-7) | 126,300 (11.7) | 9.50 (18.8) |
| 800 mg o.d. | 18,700 (14.0) | 3.5 (1-7) | 268,400 (10.3) | 12.3 (22.9) |
| 1200 mg o.d. | 25,500 (10.8) | 3 (0.5-6) | 423,000 (10.9) | 13.1 (20.1) |
| 1800 mg o.d. | 47,700 (23.3) | 2 (0.5-4) | 740,300 (19.6) | 11.3 (28.8) |
| 2400 mg o.d. | 56,500 (20.0) | 2 (1.5-8) | 905,900 (12.8) | 10.4 (24.1) |

| | | | | |
|---|---|---|---|---|
| CV = Coefficient of variation (%); Cₘₐₓ = Maximum plasma concentration; AUC₀₋₂₄ₕ = Area under the curve of plasma concentration-time over 24 h; tₘₐₓ = Time to Cₘₐₓ; t_{1/2} = Elimination half-life | | | | |

### STUDY DISCUSSION

Once-daily administration of eslicarbazepine acetate was found to be more efficacious than the same total dosage divided into twice-daily doses, and is clearly more efficacious in reducing epileptic seizures than placebo. 800 mg and 1200 mg once-daily doses of eslicarbazepine acetate were shown to be noticeably more efficacious in reducing epileptic seizures than twice-daily doses achieving the same total daily dosage.

Eslicarbazepine acetate was shown to be extensively metabolized to eslicarbazepine and, in a minor extent, to R-licarbazepine. Eslicarbazepine represented between 95% and 98% of total systemic drug exposure (as assessed by AUC_{0-τ}, i.e., AUC over the dosing interval) and, therefore, is believed to be mainly responsible for pharmacological activity following administration of eslicarbazepine acetate. Plasma concentrations of parent drug (eslicarbazepine acetate) were systematically found to be below the limit of quantification. With multiple-dosing, steady-state plasma concentrations were attained at 4 to 5 days of administration in both groups, consistent with an effective half-life on the order of about 20-24 hours.

The kinetic profile of eslicarbazepine in the once-daily group was markedly different from the twice-daily group with statistical differences found for some of the pharmacokinetic parameters assessed (Cₘₐₓ, AUC₀₋ₜ and AUC_{0-∞}) following multiple oral dosing of eslicarbazepine acetate. In fact, the total exposure of healthy volunteers to eslicarbazepine in the once-daily group was unexpectedly at least 26% higher than in the twice-daily group. This unexpected result is in line with finding in epileptic patients that once-daily administration of eslicarbazepine acetate was more efficacious than the same total daily dosage divided into twice-daily doses. Though this result might imply that the enhanced clinical efficacy would result from an increase in the rate (Cₘₐₓ) and extent (AUC) of exposure to eslicarbazepine, the reasons for such an enhanced extent of exposure after once-daily versus twice-daily administration remain unexplained.

In addition to the exemplary aspects and embodiments described above, further aspects and embodiments will become apparent to those skilled in the art by study of the foregoing description. It will be appreciated that the invention may be modified within the scope of the amended claims.

## Claims

1. The use of eslicarbazepine acetate in the manufacture of a pharmaceutical composition for treating post-herpetic neuralgia.

2. The use according to claim 1, wherein the pharmaceutical composition is for once-daily administration.

3. The use according to claim 2, wherein the once-daily dose is administered in an amount resulting in a maximum observed plasma concentration, Cₘₐₓ, of eslicarbazepine greater than about 7,400 ng/mL.

4. The use according to claim 3, wherein the once-daily dose is administered in an amount resulting in a Cₘₐₓ of eslicarbazepine greater than about 12,000 ng/mL.

5. The use according to claim 2, wherein the once-daily dose is administered in an amount resulting in an area under the concentration curve, AUC_{0-τ}, of eslicarbazepine greater than about 111,000 ng·h/mL, wherein τ is the dosing interval.

6. The use according to claim 5, wherein the once-daily dose is administered in an amount resulting in a AUC_{0-τ} of eslicarbazepine greater than about 240,000 ng·h/mL.

7. The use according to any one of claims 2 to 5, wherein the once-daily dose is administered in a dosage comprising at least about 400 mg of eslicarbazepine acetate.

8. The use according to claim 6, wherein the once-daily dose is administered in a dosage comprising at least 800 mg of eslicarbazepine acetate.

9. The use according to any preceding claim, wherein the active ingredient in the pharmaceutical composition consists essentially of eslicarbazepine acetate.

10. The use according to any preceding claim, wherein the pharmaceutical composition is formulated for oral administration.

11. The use according to any preceding claim, wherein the pharmaceutical composition is in tablet form.

12. The use according to any one of claims 1 to 10, wherein the pharmaceutical composition is in oral suspension form.

13. The use according to claim 12, wherein the pharmaceutical composition comprises at least one excipient, auxiliary substance and/or carrier material.

14. The use according to claim 13, wherein the at least one excipient, auxiliary substance and/or carrier material is selected from xanthan gum, macrogol stearate, methylparaben, propylparaben, saccharin sodium, sorbitol, buffers, flavorings and combinations thereof.

## Patentansprüche

1. Verwendung von Eslicarbazepin-Acetat bei der Herstellung einer pharmazeutischen Zusammensetzung zur Behandlung von postherpetischer Neuralgie.

2. Verwendung nach Anspruch 1, wobei die pharmazeutische Zusammensetzung zur einmal täglichen Verabreichung ist.

3. Verwendung nach Anspruch 2, wobei die einmal tägliche Dosis in einer Menge verabreicht wird, die zu einer maximal beobachteten Plasmakonzentration, Cₘₐₓ, von Eslicarbazepin von mehr als etwa 7.400 ng/ml führt.

4. Verwendung nach Anspruch 3, wobei die einmal tägliche Dosis in einer Menge verabreicht wird, die zu einer Cₘₐₓ von Eslicarbazepin von mehr als etwa 12.000 ng/ml führt.

5. Verwendung nach Anspruch 2, wobei die einmal tägliche Dosis in einer Menge verabreicht wird, die zu einer Fläche unter der Konzentrationskurve, AUC_{0-τ}, von Eslicarbazepin von mehr als etwa 111.000 ng·h/ml führt, wobei τ das Dosierungsintervall ist.

6. Verwendung nach Anspruch 5, wobei die einmal tägliche Dosis in einer Menge verabreicht wird, die zu einer AUC_{0-τ} von Eslicarbazepin von mehr als etwa 240.000 ng·h/ml führt.

7. Verwendung nach einem der Ansprüche 2 bis 5, wobei die einmal tägliche Dosis in einer Dosierung verabreicht wird, die mindestens etwa 400 mg Eslicarbazepin-Acetat umfasst.

8. Verwendung nach Anspruch 6, wobei die einmal tägliche Dosis in einer Dosierung verabreicht wird, die mindestens 800 mg Eslicarbazepin-Acetat umfasst.

9. Verwendung nach einem vorhergehenden Anspruch, wobei der Wirkstoff in der pharmazeutischen Zusammensetzung im Wesentlichen in Eslicarbazepin-Acetat besteht.

10. Verwendung nach einem vorhergehenden Anspruch, wobei die pharmazeutische Zusammensetzung zur oralen Verabreichung formuliert ist.

11. Verwendung nach einem vorhergehenden Anspruch, wobei die pharmazeutische Zusammensetzung in Tablettenform ist.

12. Verwendung nach einem der Ansprüche 1 bis 10, wobei die pharmazeutische Zusammensetzung in oraler Suspensionsform ist.

13. Verwendung nach Anspruch 12, wobei die pharmazeutische Zusammensetzung mindestens ein Vehikel, einen Hilfsstoff und/oder einen Trägerstoff umfasst.

14. Verwendung nach Anspruch 13, wobei das mindestens eine Vehikel, der mindestens eine Hilfsstoff und/oder der mindestens eine Trägerstoff aus Xanthan, Macrogolstearat, Methylparaben, Propylparaben, Saccharin-Natrium, Sorbit, Puffern, Geschmacksstoffen und Kombinationen davon ausgewählt ist.

## Revendications

1. Utilisation d'acétate d'eslicarbazépine pour la fabrication d'une composition pharmaceutique destinée au traitement de la névralgie post-herpétique.

2. Utilisation selon la revendication 1, dans laquelle la composition pharmaceutique est destinée à une administration quotidienne unique.

3. Utilisation selon la revendication 2, dans laquelle la dose quotidienne unique est administrée en une quantité menant à une concentration plasmatique maximale observée, Cₘₐₓ, d'eslicarbazépine supérieure à environ 7400 ng/mL.

4. Utilisation selon la revendication 3, dans laquelle la dose quotidienne unique est administrée en une quantité menant à une Cₘₐₓ d'eslicarbazépine supérieure à environ 12 000 ng/mL.

5. Utilisation selon la revendication 2, dans laquelle la dose quotidienne unique est administrée en une quantité menant à une aire sous la courbe de concentration, AUC_{0-T}, d'eslicarbazépine supérieure à environ 111 000 ng·h/mL, où T est l'intervalle posologique.

6. Utilisation selon la revendication 5, dans laquelle la dose quotidienne unique est administrée en une quantité menant à une AUC_{0-T} d'eslicarbazépine supérieure à environ 240 000 ng·h/mL.

7. Utilisation selon l'une quelconque des revendications 2 à 5, dans laquelle la dose quotidienne unique est administrée selon une posologie comprenant au moins environ 400 mg d'acétate d'eslicarbazépine.

8. Utilisation selon la revendication 6, dans laquelle la dose quotidienne unique est administrée selon une posologie comprenant au moins 800 mg d'acétate d'eslicarbazépine.

9. Utilisation selon l'une quelconque des revendications précédentes, dans laquelle le principe actif dans la composition pharmaceutique consiste essentiellement en acétate d'eslicarbazépine.

10. Utilisation selon l'une quelconque des revendications précédentes, dans laquelle la composition pharmaceutique est formulée pour une administration par voie orale.

11. Utilisation selon l'une quelconque des revendications précédentes, dans laquelle la composition pharmaceutique est sous forme de comprimé.

12. Utilisation selon l'une quelconque des revendications 1 à 10, dans laquelle la composition pharmaceutique est sous forme de suspension orale.

13. Utilisation selon la revendication 12, dans laquelle la composition pharmaceutique comprend au moins un excipient, une substance auxiliaire et/ou un véhicule.

14. Utilisation selon la revendication 13, dans laquelle le ou les excipients, substances auxiliaires et/ou véhicules sont choisis parmi dans le groupe consistant en gomme de xanthane, stéarate de macrogol, 4-hydroxybenzoate de méthyle, p-hydroxybenzoate de propyle, saccharine sodique, sorbitol, tampons, aromatisants et leurs combinaisons.
